# EUROPÄISCHE PATENTSCHRIFT

(11) **EP 0 796 080 B1**
(45) Veröffentlichungstag und Bekanntmachung des Hinweises auf die Patenterteilung: **11.11.1998**
(21) Anmeldenummer: 95939228.3
(22) Anmeldetag: 07.12.1995
(51) Int. Cl.: A61K 7/06

(54) **KOMBINATIONSPRÄPARAT ZUR FÖRDERUNG DES HAARWACHSTUMS UND GGF. DES HAUT- UND NAGELWACHSTUMS SOWIE ZUR VERHINDERUNG BZW. ZUR BESEITIGUNG VON HAARAUSFALL**
COMBINED PREPARATION FOR STIMULATING HAIR GROWTH AND OPTIONALLY NAIL GROWTH AND SKIN REGENERATION AND FOR PREVENTING OR COMBATTING HAIR LOSS
PREPARATION COMBINEE POUR STIMULER LA POUSSE DES CHEVEUX ET EVENTUELLEMENT CELLE DES ONGLES ET LA REGENERESCENCE DES TISSUS CUTANES, AINSI QUE POUR EMPECHER OU LUTTER CONTRE LA CHUTE DES CHEVEUX

(30) Priorität: 08.12.1994 DE 4443585; 15.03.1995 DE 19509354
(43) Veröffentlichungstag der Anmeldung: 24.09.1997
(73) Patentinhaber: Klett-Loch, Lore Maria, 68309 Mannheim (DE)
(72) Erfinder: Klett-Loch, Lore Maria, 68309 Mannheim (DE)
(74) Vertreter: Naumann, Ulrich, Dr.-Ing.
(86) Internationale Anmeldenummer: DE9501745
(87) Internationale Veröffentlichungsnummer: WO9617584

(56) Entgegenhaltungen:
- DE-A- 2 331 456
- DE-A- 3 417 136
- FR-A- 2 704 394
- US-A- 4 863 950
- DATABASE WPI Section Ch, Week 9429 Derwent Publications Ltd., London, GB; Class D21, AN 94-236932 XP002006437 & ES,A,2 052 450 (RUBIO CERDA L) , 1.Juli 1994
- PATENT ABSTRACTS OF JAPAN vol. 007, no. 014 (C-146), 20.Januar 1983 & JP,A,57 171908 (SHIZUYA SHIOZU), 22.Oktober 1982,
- PATENT ABSTRACTS OF JAPAN vol. 008, no. 015 (C-206), 21.Januar 1984 & JP,A,58 183614 (SHISEIDO KK), 26.Oktober 1983,
- PATENT ABSTRACTS OF JAPAN vol. 008, no. 085 (C-219), 18.April 1984 & JP,A,59 007111 (SHISEIDO KK), 14.Januar 1984,
- CHEMICAL ABSTRACTS, vol. 114, no. 2, 1991 Columbus, Ohio, US; abstract no. 11983w, "Hair preparations for preventing hair shedding in nonhuman mamals" Seite 336; XP002006435 & IL,A,86 321 (ELAD AND EINAT ENTERPRISES)
- PATENT ABSTRACTS OF JAPAN vol. 001, no. 157 (C-032), 14.Dezember 1977 & JP,A,52 099223 (EISAI CO LTD), 19.August 1977,
- CHEMICAL ABSTRACTS, vol. 100, no. 22, 1984 Columbus, Ohio, US; abstract no. 179953w, "Hair preparaitons ocntaining ubiquinones and peripheral vasodilators" Seite 313; XP002006436 & JP,A,05 913 710 (SHISHEIDO CO. LTD.)

## Beschreibung

Die Erfindung betrifft ein Kombinationspräparat einerseits zur Förderung des Haarwachstums und ggf. des Haut- und Nagelwachstums und andererseits zur Verhinderung von Haarausfall bzw. zur Beseitigung von Haarausfall insoweit, als die Haarfollikel noch vorhanden bzw. intakt sind. Genauergesagt handelt es sich hier um ein Kombinationspräparat mit einer Vitamine, Enzyme und Aminosäuren enthaltenden Wirkstoffkombination.

Präparate zur Förderung des Haarwachstums sind seit langem aus der Praxis bekannt und weisen unterschiedlichste Zusammensetzungen auf. Beispielsweise können Schwefel oder Schwefelverbindungen, Vitamine, Hormone, cholesterin- und lecithinhaltige Stoffe in solchen Präparaten enthalten sein. Derartige Kombinationspräparate dienen einerseits zur vorbeugenden Gesunderhaltung und Pflege des Haares, andererseits aber auch zur Heilung von Haarausfall.

Sowohl bei Frauen als auch bei Männern geht das Auftreten eines gesteigerten Haarausfalls mit der Angst vor einer völligen Glatzenbildung einher. Neben dem medizinischen Aspekt stellen Haarwachstumsstörungen für den Betroffenen somit ein großes persönliches Problem dar. Die Wachstumsgeschwindigkeit des Haars beträgt ca. 0,35 mm/Tag, die Haardichte etwa 80.000 bis 150.000 Haare/Kopf. Bei einem Haarverlust von 100 Haaren/Tag liegt bereits ein pathologisches Effluvium vor.

Aus intakt gebliebenen Haarfollikeln können Haare wieder nachwachsen. Während des mehrphasigen, langwierigen Nachwachsens kann es jedoch zur Schrumpfung der Haarfollikel und dabei zum allmählichen Haarausfall kommen.

Seit einigen Jahren ist ein GKL-Wirkstoffkomplex der Anmelderin auf dem Markt, dessen Wirkstoffkombination mit einem hohen Anteil an Aminosäuren, Enzymen und den Vitaminen B, E, F angereichert ist. In besonderem Maße sind in dem bekannten Kombinationspräparat Kälberthymusextrakt, pflanzliche Extrakte und tierische Öle enthalten. Der GKL-Wirkstoffkomplex ist von zahlreichen Kliniken und Instituten durch Behandlung verschiedener Alopezieformen mit Erfolg getestet worden. Eine der zahlreichen Veröffentlichungen erfolgte bspw. in der Fachzeitschritt "Der Onkologe" (1/90) oder in dem Sonder-druck der Fachzeitschrift "Der Deutsche Dermatologe" (11/93).

Insgesamt ist in mehreren Tests ermittelt worden, daß der GKL-Wirkstoffkomplex bei allen Arten von Haarausfall bei Männem und Frauen wirksam ist, androgenetischem Haarausfall vorbeugt, Glatzenbildung sowie Haarausfall bei Krebspatienten unter leichter bis mittelschwerer Chemotherapie verhindert und darüber hinaus auch Kopfschuppenbildung entgegensteht.

Die Anwendung bekannter Haarwuchsmittel im allgemeinen und des GKL-Wirkstoffkomplexes im besonderem erfolgt in der Regel äußerlich. Die Darreichungsformen der bekannten Haarwuchsmittel sind flüssig, wobei die fließfähigen Produkte in Form von Emulsionen, Lotionen, Shampoos, Haarkuren oder - wässern angeboten werden.

Des weiteren sind - für sich gesehen - Nahrungsergänzungsmittel bekannt, die zur günstigen Beeinflussung des Stoffwechsels des menschlichen Körpers dienen. Hierbei können zielgerichtete Wirkungen erreicht werden. Nahrungsergänzungsmittel, die auf eine diätetische Anwendung abzielen, können beispielsweise aufgrund ihrer Zusammensetzung einen Oxidationsschutz durch Bindung der gerade bei einer Diät vermehrt entstehenden, freien Radikale bewirken. Die Nahrungsergänzungsmittel werden im Körperinneren, insbesondere durch Resorption körpereigener Zellen, wirksam.

Ausgehend von diesen, an sich völlig unterschiedlichen, jedoch einen Themenkomplex bildenden Themenkreisen liegt der vorliegenden Erfindung die Aufgabe zugrunde, ein Kombinationspräparat der in Rede stehenden Art anzugeben, das seine Wirkung vom Körperinneren her in den Körperzellen entfaltet. Zur Steigerung der Wirksamkeit des in Rede stehenden Kombinationspräparates, zumindest im Hinblick auf die Förderung des Haarwachstums, wird außerdem eine Verwendung des Kombinationspräparates angegeben.

Die voranstehende Aufgabe wird durch die Merkmale der Patentansprüche 1 und 29 gelöst.

Gemäß den Merkmalen des Patentanspruches 1 ist das erfindungsgemäße Kombinationspräparat derart ausgestaltet, daß seine Wirkstoffkombination Bestandteil einer oral oder auch topisch verabreichbaren Darreichungsform ist und daß die Wirkstoffkombination in etwa folgende Stoffe enthält:

| | | |
|---|---|---|
| Provitamin A | 1,25 | Gew.-%, |
| Vitamin B₁ | 0,27 | Gew.-%, |
| Vitamine der B₂-Gruppe | 5,25 | Gew.-%, |
| Vitamin B₆ | 0,37 | Gew.-%, |
| Vitamin B₁₂ | 0,001 | Gew.-%, |
| Vitamin C | 15,61 | Gew.-%, |
| Vitamin E | 2,5 | Gew.-%, |
| Coenzym Q 10 | 1,04 | Gew.-%, |
| Methionin | 0,02 | Gew.-% und |
| Cystin | 0,018 | Gew.-%. |

Gemäß den Merkmalen des Patentanspruches 29 erfolgt die Verwendung des beanspruchten Kombinationspräparates in Ergänzung zu einem bereits topisch anwendbaren Haarwuchsförderungsmittel, insbesondere zu einem thymushaltigen Therapeutikum.

Das zuvor bereits erwähnte Thymuspräparat mit dem GKL-Wirkstoffkomplex wird ausschließlich äußerlich angewandt. Ausgehend von der grundsätzlichen Wirkung bekannter Nahrungsergänzungsmittel im Körperinneren ist nun erkannt worden, daß eine gezielte Beeinflussung körpereigener, mit dem Haar-, Haut- und Nagelwachstum in Zusammenhang stehender Zellen möglich ist.

Erfindungsgemäß kann nun ein haar-, haut- und nagelwachstumsförderndes Präparat bzw. ein den Haarausfall verhinderndes oder beseitigendes Präparat im Inneren des Körpers seine Wirkung entfalten, wenn es einerseits eine die Körperzellen beeinflussende Zusammensetzung aufweist und andererseits in einer in den Körper einführbaren Form vorliegt. Auf diese Weise läßt sich nämlich die Aktivität der Haarfollikel in der Unterhaut des Menschen von Innen her über die Resorption von Wirkstoffe beeinflussen. Besonders der mehrphasige, langwierige Nachwachsprozeß läßt sich unter weitgehender Vermeidung einer Schrumpfung der Haarfollikel beschleunigen. Von wesentlicher Bedeutung ist auch die vorbeugende Wirkung des erfindungsgemäßen Kombinationspräparates im Hinblick auf die Gesunderhaltung des Haares, die mit einer grundsätzlichen Verbesserung der Haarstruktur einhergeht.

Als erfindungswesentlich ist weiter erkannt worden, daß die Wirksamkeit des Kombinationspräparats im Hinblick auf die Förderung des Haarwachstums bzw. Verhinderung des Haarausfalls erheblich gesteigert werden kann, wenn es in Ergänzung zu äußerlich anwendbaren Haarwuchsmitteln - oral - verwendet wird, wobei auch hier eine rein topische Anwendung möglich ist. Besonders im Hinblick auf die Anwendung des in der Beschreibungseinleitung beschriebenen, klinisch getesteten GKL-Wirkstoffkomplexes kann eine hohe Wirksamkeit des Kombinationspräparates erzielt werden. Letztlich erfolgt bei der erfindungsgemäßen Verwendung sowohl eine äußerliche als auch eine innerliche Behandlung der betroffenen Körperzellen. An dieser Stelle sei besonders hervorgehoben, daß auch das thymushaltige Therapeutikum auf die Haut oder die Nägel auftragbar ist, so daß auch bezüglich dieser Indikationen von einer erhöhten Wirksamkeit ausgegangen werden kann.

Im Hinblick auf die Zusammensetzung der Wirkstoffkombination ist nun weiter erkannt worden, daß das Zellwachstum an den zu behandelnden Bereichen des Körpers positiv beeinflußt werden kann, wenn die zugeführte Energie optimal verwertet wird. Eine optimale Verwertung der zugeführten Energie wird durch das Zusammenwirken unterschiedlicher Vitamine einerseits und ernährender Bestandteile andererseits in hohem Maße gefördert. Das Coenzym Q 10 ist beispielsweise besonders geeignet, die Energiegewinnung in der Zelle zu verbessern, da es sich hierbei um ein sog. Hilfssubstrat der Atmungskette handelt. Coenzym Q 10 ist in der Lage Elektronen sowohl aufzunehmen als auch abzugeben und diese zwischen den sog. Cytochromen und Flavoproteinen auszutauschen. Alternativ kommen weitere Substanzen als Wirkstoffe in Betracht, da diese für die Energiegewinnung in der Zelle wesentliche Funktionen begünstigen.

Nach einer besonders vorteilhaften Zusammensetzung des Kombinationspräparates werden in der Wirkstoffkombination verschiedene Vitamine der B₂-Gruppe angewendet. In besonderem Maße werden Nicotinamid, Pantothensäure und Riboflavin in der Wirkstoffkombination berücksichtigt.

Als besonders vorteilhaft hat es sich erwiesen, die Vitamine der B₂-Gruppe zu folgenden Anteilen in der Wirkstoffkombination zu berücksichtigen:

| | | |
|---|---|---|
| Riboflavin | 0,35 | Gew.-%, |
| Nicotinamid | 3,75 | Gew.-%, |
| Folsäure | 0,09 | Gew.-%, |
| Pantothensäure | 1,04 | Gew.-%, |
| Biotin | 0,02 | Gew.-%., |

wobei einzelne Bestandteile in ihrer Dosis erhöht oder verringert werden können.

Von ganz besonderem Vorteil ist die Wirkung der Pantothensäure, die für sich gesehen, zur Behandlung von Haardepigmentierungen und allgemeinen Hautfunktionsstörungen verwendet wird und darüber hinaus die Wundheilung fördert. Besonders bevorzugt wird bei dem erfindungsgemäßen Kombinationspräparat Pantothensäure aus Calciumpantothenat eingesetzt.

Als weiterer Bestandteil der Wirkstoffkombination wird Provitamin A verwendet, wobei es sich bevorzugt um beta-Carotin handelt. Alternativ könnten auch Mischformen des Provitamins A aus alpha-, beta- oder gamma-Carotin und anderen Carotinoiden vorliegen. Das Provitamin A wird jedenfalls im Organismus verändert, der die eigentlichen Wirkstoffe herstellen kann. Das Provitamin A könnte wiederum aus pflanzlichen Rohstoffen gewonnen sein. Als alpha- und beta-Carotinlieferanten kommen beispielsweise Rüben bzw. Karotten in Betracht.

Das in der Wirkstoffkombination vorgesehene Vitamin B₁ wird vorzugsweise in Form eines Salzes, nämlich als Thiaminnitrat, in die Rezeptur eingebracht. Auch das in der Wirkstoffkombination enthaltene Vitamin B₆ liegt in Form eines Salzes, nämlich als Pyridoxinhydrochloride vor. Das Vitamin B₆, das auch als Pyridoxin bezeichnet wird, ist insoweit von ganz besonderer Bedeutung, daß bei Unterversorgung mit Vitamin B₆ entzündliche Hautveränderungen auftreten können. Sowohl bei dem Vitamin B₁ als auch bei dem Vitamin B₆ könnten auch andere Salze oder die Reinformen eine Rolle spielen.

Im Hinblick auf eine therapeutische Anwendbarkeit des Vitamins B₁₂ wird dieses in Form von Cyanocobalamin verwendet. Bei Cyanocobalamin wird der Ligand am Kobalt-Zentralatom der Verbindung durch eine CN-Gruppe gebildet. Anstelle des Vitamins B₁₂ wäre es auch denkbar, Vitamin-B₁₂-Analoge einzusetzen.

Nach einer besonders bevorzugten Ausgestaltung ist das Vitamin B₁₂ in Form einer Zubereitung mit Gelatine vorgesehen. Der Vorteil der Verwendung einer Zubereitung aus Vitamin B₁₂ und Gelatine gegenüber der Verwendung des Vitamins B₁₂ in Reinsubstanz liegt in der Handhabung und in der Beständigkeit des Cyanocobalamins gegenüber Licht und Luftfeuchtigkeit. Wird die das Vitamin B₁₂ enthaltende Gelatine-Zubereitung in der Wirkstoffkombination eingesetzt, so stimmt der prozentuale Anteil des Vitamins B₁₂ in der Gelatine mit dem tatsächlich erforderlichen Gehalt an Vitamin B₁₂ in der Wirkstoffkombination überein. Die Vitamin B₁₂-Gelatine-Zubereitung beträgt in der Wirkstoffkombination insgesamt etwa 1,4 Gew.-%.

Einen besonderen Stellenwert nimmt das Vitamin C in der Wirkstoffkombination ein. Vorzugsweise wird Vitamin C aus Calziumascorbat eingesetzt. Calziumascorbat ist ein Calziumsalz der Ascorbinsäure und im Handel erhältlich. Die besondere Bedeutung des Vitamins C ergibt sich insbesondere daraus, daß die Ascorbinsäure das Zellwachstum - also auch das Wachstum der Haare ausgehend von dem Haarfollikel - aktiviert.
Im Hinblick auf die stärkste Wirksamkeit wird bei dem Wirkstoffkombinationsbestandteil Vitamin E eine Alpha-Tocopherol-Verbindung bevorzugt. Das D,L-alpha-Tocopheralacetat ist im Handel in synthetisch hergestellter Form erhältlich. Der Präfix "D, L" weist darauf hin, daß die Acetatverbindung an bestimmter Stelle racemisch, d.h. optisch aktiv, ist. Das D,L-alpha-Tocopheralacetat wird gerade aufgrund seiner optisch aktiven Konfiguration bevorzugt eingesetzt. Denkbar wäre eventuell die Verwendung von alpha-Tocopherol aus natürlichem, pflanzlichen oder tierischen Material.

Die beiden in der Wirkstoffkombination vorkommenden Aminosäuren Methionin und Cystin werden bevorzugt in bestimmten optisch aktiven Konfigurationen verwendet. So handelt es sich einerseits um racemisches Methionin und andererseits um L-Cystin. Werden Methionin und Cystin als Reinsubstanzen in der Wirkstoffkombination eingesetzt, machen ihre Anteile in der Wirkstoffkombination jeweils 0,02 Gew.-% aus.

Die beiden Aminosäuren könnten nun Bestandteil eines Getreide- oder Pflanzenproteins sein. Ganz besonders bevorzugt werden Methionin und Cystin, jedoch als Bestandteile eines Milchproteins, in die Wirkstoffkombination eingeführt.

Als besonders vorteilhaft hat sich die Verwendung von Lactalbumin 80 herausgestellt. Lactalbumin weist einen Proteingehalt von 80 % auf. Der Rest der Zubereitung besteht aus Milchzucker, Fett, Mineralien und Wasser. Der Gehalt an Cystin bzw. Methionin in Lactalbumin 80 beträgt 3,6 bzw. 4 %. In der Gewichtseinheit mg heißt das, 3,6 % Cystin entsprechen 0,0864 mg und 4 % Methionin entsprechen 0,096 mg. Durch einen Anteil von 0,62 Gew.-% Lactalbumin 80 in der Wirkstoffkombination sind in dieser 0,028 Gew.-% Methionin und 0,018 Gew.-% Cystin enthalten. Über die Verwendung von Lactalbumin 80 kann der Anteil von jeweils 0,02 Gew.-% Cystin bzw. Methionin in Reinsubstanz fast vollständig substituiert werden.

Zur Verabreichung des Kombinationspräparats können verschiedene Darreichungsformen gewählt werden. Neben Tabletten oder Dragees werden vor allem Kapseln bevorzugt, die mit der Wirkstoffkombination gefüllt werden. Das Verfüllen der Wirkstoffkombination kann nun in vorteilhafter Weise dadurch optimiert werden, daß die Wirkstoffkombination zusammen mit Leinöl eine Zubereitung bildet. Außer der Überführung der Zubereitung in eine pastöse Form hat Leinöl die Aufgabe, essentielle Fettsäuren in das Kombinationspräparat und schließlich in den menschlichen Körper einzuführen und die Haut, die Haare und die Nägel mit Nährsubstanzen zu versorgen.

Des weiteren kann das erfindungsgemäße Kombinationspräparat weitere pflanzliche Öle oder Fette als Nähr- und Trägersubstanz enthalten, die sowohl unverändert als auch hydriert sind. Auch diese pflanzlichen Öle oder Fette dienen einerseits dazu, die Verfüllbarkeit der Zubereitung zu gewährleisten und andererseits, essentielle Fettsäuren in das Kombinationspräparat einzuführen. Neben unverändertem Leinöl hat es sich als vorteilhaft erwiesen, hydriertes Sojabohnenöl und/oder unverändertes Erdnußöl zu verwenden.

Damit nun die zum Teil wasserhaltigen oder pulvrigen Substanzen der Wirkstoffkombination in der öligen Zubereitung subspendierbar gemacht werden können, wird in vorteilhafter Weise ein Emulgator zugesetzt. Der Emulgator könnte beispielsweise eine Mischung aus Sojalecithin und Sojaphosphatiden umfassen, die im Lebensmittelbereich bereits zur besseren Löslichkeit von Pulver in Flüssigkeiten verwendet wird.

Die voranstehend beschriebene Erstellung der Zubereitung, nämlich die Überführung der bisher erörterten Bestandteile des Kombinationspräparats in einen pastösen Zustand, zielt auf die Verwendung von Kapseln als Darreichungsform ab. Bevorzugt werden hier Weichgelatinekapseln verwendet.

Die zur Erstellung der Kapsel notwendigen Hauptbestandteile werden durch Gelatine und Glycerol repräsentiert. Glycerol ist vorzugsweise zu 85 Gew.-% in der ungefüllten Kapsel enthalten. Des weiteren enthält die Kapsel ein unverändertes Erdnußöl als Weichmacher. Darüber hinaus kann ein Stabilisator vorgesehen werden, der eine konservierende Funktion ausübt. Zur Erstellung der Weichgelatinekapsel hat sich insbesondere der Stabilisator 420 als geeignet erwiesen.

Der Gelatinemasse können darüberhinaus auch Farbstoffe (Lebensmittelfarben) und/oder Trübungsmittel (z. B. Titandioxid) zugesetzt werden. Von ganz besonderem Vorteil ist der Einsatz von beta-Carotin als Färbemittel. Auf diese Weise wird das auf die Körperzelle wirkende beta-Carotin auch als Kapselbestandteil in den Körper eingeführt.

Nach einem bevorzugten Ausführungsbeispiel weist eine Kapsel ein Gewicht von 480 mg auf. Aus den Merkmalen des Patentanspruches 28 ergibt sich die gewichtsmäßige Verteilung der Bestandteile der Wirkstoffkombination und der übrigen Stoffe auf das Gesamtgewicht von 480 mg der Kapsel. Hieraus wird ersichtlich, daß das Eigengewicht der Kapsel das Füllgewicht geringfügig unterschreitet. Hierdurch ist eine ausreichende Stabilität der Kapselwandung gewährleistet.

Nach dem hier gewählten Ausführungsbeispiel enthält eine Kapsel mit einem Gesamtgewicht von 480 mg durchschnittlich 0,15 mg verwertbare Kohlehydrate, 157,42 mg verwertbare Fette und 98,46 mg verwertbare Eiweißstoffe. Der durchschnittliche physiologische Brennwert beträgt pro Kapsel etwa 1,9 kcal zw. 7,88 kJ.

Des weiteren ist es von Vorteil, die Wirkstoffkombination um den Bestandteil Selen bzw. Selen-Hefe zu ergänzen, wobei hier ein Anteil von bis zu 5 Gew.-% der gesamten Wirkstoffkombination denkbar ist. Bei Selen handelt es sich um einen Bestandteil des Enzyms Glutathion-Peroxidase, das in den Erythrozyten (rote Blutkörperchen) vorkommt und sogenannte ungesättigte Zellmembramlipide vor dem Angriff des Sauerstoffmoleküls und anderer freier Radikale schützt. Selen gehört daher zu den essentiellen Elementen. Eine Darreichung von Selen als Selen-Hefe ist von Vorteil, da das Selen durch Begleitstoffe der Hefe bes-ser resorbiert werden kann. Außerdem enthält die Hefe selbst noch einen relativ hohen Anteil des Vitamin B-Komplexes.

Als weiteren Bestandteil kann die Wirkstoffkombination ein Thymus-Extrakt umfassen. Dabei kann es sich sowohl um ein Extrakt aus Rinderthymus als auch um synthetisch hergestellte Thymus-Peptide handeln, die bevorzugt in sprühgetrockneter Form beigemengt werden. Im Falle des Thymus-Extraktes könnte ein Gewichtsanteil von bis zu 50 % der gesamten Wirkstoffkombination vorgesehen sein. Im Falle der Thymus-Peptide läßt sich dieser Anteil verringern, so daß von einem Gewichtsanteil von bis zu 5 Gew.-% auszugehen ist. Dabei ist von ganz besonderer Bedeutung, daß der Zusatz eines Thymus-Extrakts zur oralen Appliaktionsform die Wirkung einer äußerlichen Applikation unterstützt, wobei die äußerliche Applikation mit dem eingangs genannten Produkt "Thymu-Skin" erfolgen kann.

Bei der Verwendung synthetischer Thymus-Peptide ist wesentlich, daß diese aus dem Gesamtextrakt isoliert und identifiziert werden. Entsprechend lassen sich diese Peptide - in synthetischer Form - dem Präparat zusetzen. Die Peptide haben qualitativ die gleiche Wirkung wie das Thymusextrakt selbst, schließen jedoch unwirksame Begleitstoffe aus und können in geringer Menge eingesetzt werden.

Ergänzend sei darauf hingewiesen, daß das Kombinationspräparat bzw. die Wirkstoffkombination den Wirkstoff "Melatonin" enthalten kann, in dem man altersverzögernde Eigenschaften, eine Verbesserung der Immunabwehrkräfte und die Wirkung als Katalysator zur Beeinflussung biologischer Prozesse zuschreibt. Auch hier ist von einer Begünstigung des Wachstums von Haaren, Fingernägeln und Haut auszugehen. Dieser Wirkstoff Melatonin kann in einem Spektrum von 0,5 bis etwa 30 mg vorgesehen sein.

Nachfolgend findet sich eine Aufzählung einiger der zuvor beschriebenen Wirkstoffe mit Nomenklaturangaben:

Vitamin B1 als Thiaminnitrat, DAB, Ph.Eur., USP; Vitamin B2 als Riboflavin, DAB, Ph.Eur., USP; Nicotinamid, DAB, Ph.Eur.; Folsäure, DAB, Ph.Eur., USP; Pantothensäure aus Calciumpantothenat, DAB, Ph.Eur., USP; Vitamin B6 als Pyridoxinhydrochlorid, DAB, Ph.Eur., USP; Vitamin C aus Calciumascorbat, FCC; racemisches Methionin, DAB, Ph.Eur., USP XXI und L-Cystin, FCC.

## Patentansprüche

1. Kombinationspräparat zur Förderung des Haarwachstums und ggf. des Haut- und Nagelwachstums sowie zur Verhinderung und Beseitigung von Haarausfall mit einer Vitamine, Enzyme und Aminosäuren enthaltenden Wirkstoffkombination,
**dadurch gekennzeichnet**, daß die Wirkstoffkombination Bestandteil einer oral oder auch topisch verabreichbaren Darreichungsform und folgende Stoffe in etwa enthält:
| | | |
|---|---|---|
| Provitamin A | 1,25 | Gew.-%, |
| Vitamin B₁ | 0,27 | Gew.-%, |
| Vitamine der B₂-Gruppe | 5,25 | Gew.-%, |
| Vitamin B₆ | 0,37 | Gew.-%, |
| Vitamin B₁₂ | 0,001 | Gew.-%, |
| Vitamin C | 15,61 | Gew.-%, |
| Vitamin E | 2,5 | Gew.-%, |
| Coenzym Q 10 | 1,04 | Gew.-%, |
| Methionin | 0,02 | Gew.-% und |
| Cystin | 0,018 | Gew.-%. |

2. Kombinationspräparat nach Anspruch 1, dadurch gekennzeichnet, daß es sich bei den Vitaminen der B₂-Gruppe hauptsächlich um Nicotinamid, Pantothensäure und Riboflavin und ggf. um Folsäure und Biotin handelt und daß die Vitamine der B₂-Gruppe vorzugsweise zu folgenden Anteilen in der Wirkstoffkombination enthalten sind:
| | | |
|---|---|---|
| Riboflavin | 0,35 | Gew.-%, |
| Nicotinamid | 3,75 | Gew.-%, |
| Folsäure | 0,09 | Gew.-%, |
| Pantothensäure | 1,04 | Gew.-%, |
| Biotin | 0,02 | Gew.-%, |
und wobei es sich bei Pantothensäure um Pantothensäure aus Calciumpantothenat handeln kann.

3. Kombinationspräparat nach Anspruch 1 oder 2, dadurch gekennzeichnet, daß es sich bei dem Provitamin A zumindest vorwiegend um beta-Carotin handelt, daß das Vitamin B₁ vorzugsweise als Thiaminnitrat, das Vitamin B₆ vorzugsweise als Pyridoxinhydrochlorid, das Vitamin B₁₂ vorzugsweise als Cyanocobalamin und das Vitamin B₁₂ in einer Zubereitung mit Gelatine vorliegt, wobei die Zubereitung aus Vitamin B₁₂ und Gelatine vorzugsweise ca. 1,04 Gew.-% der Wirkstoffkombination ausmacht.

4. Kombinationspräparat nach einem der Ansprüche 1 bis 3, dadurch gekennzeichnet, daß es sich bei dem Vitamin C um Vitamin C aus Calciumascorbat handelt und daß das Vitamin E in Form von D,L-alpha-Tocopherolacetat vorliegt, wobei es sich bei Methionin um racemisches Methionin und bei Cystin um L-Cystin handeln kann.

5. Kombinationspräparat nach einem der Ansprüche 1 bis 4, dadurch gekennzeichnet, daß das Methionin und das Cystin als Bestandteile eines Milchproteins in der Wirkstoffkombination enthalten sind, daß es sich bei dem Milchprotein um Lactalbumin 80 handelt und daß das Lactalbumin 80 ca. 0,62 Gew.-% der Wirkstoffkombination ausmacht.

6. Kombinationspräparat nach einem der Ansprüche 1 bis 5, dadurch gekennzeichnet, daß die Wirkstoffkombination zur Erstellung der Darreichungsform in einer Zubereitung mit Leinöl vorliegt und daß das Leinöl in dem Kombinationspräparat mit einem Gewichtsanteil von 25,0 Gew.-%, enthalten ist.

7. Kombinationspräparat nach einem der Ansprüche 1 bis 6, dadurch gekennzeichnet, daß die Wirkstoffkombination zur Erstellung der Darreichungsform in einer Zubereitung mit veränderten und/oder unveränderten pflanzlichen Ölen und/oder in einer Zubereitung mit Emulgator, vorzugsweise Emulgator E 322, vorliegt.

8. Kombinationspräparat nach einem der Ansprüche 1 bis 7, dadurch gekennzeichnet, daß die Darreichungsform eine die, ggf. in Form einer Zubereitung vorliegende, Wirkstoffkombination aufnehmende Kapsel umfaßt, wobei die Kapsel zumindest Gelatine und Glyzerol sowie ein pflanzliches Öl oder Fett und/oder einen Stabilisator, insbesondere den Stabilisator 420, umfassen kann, daß die Kapsel vorzugsweise mit beta-Carotin gefärbt ist und daß die gefüllte Kapsel ein Gesamtgewicht von 480 mg aufweist, wobei das Kombinationspräparat gewichtsmäßig vorzugsweise zusammengesetzt ist aus der Wirkstoffkombination:
| | | |
|---|---|---|
| beta-Carotin | 6,0 | mg, |
| Vitamin B1 als Thiaminnitrat | 1,3 | mg, |
| Vitamin B2 als Riboflavin | 1,7 | mg, |
| Nicotinamid | 18,0 | mg, |
| Folsäure | 0,45 | mg, |
| Pantothensäure aus Calciumpantothenat | 5,0 | mg, |
| Biotin | 0,1 | mg, |
| Vitamin B6 als Pyridoxinhydrochlorid | 1,8 | mg, |
| Vitamin B12, Cyanocobalamin 0,1%-ig mit Gelatine | 5,0 | mg, |
| Vitamin C aus Calciumascorbat | 75,0 | mg, |
| Vitamin E als D,L-alpha-Tocopherolacetat | 12,0 | mg, |
| Coenzym Q 10 | 5,0 | mg, |
| Lactalbumin 80 mit 3,6% Cystin und 4 % Methionin in 80% Protein des Lactalbumin | 3,0 | mg, |
und den Hilfsstoffen:
| | | |
|---|---|---|
| Leinöl | 120,0 | mg sowie |
| Emulgator E 322 | | |
| Gelatine | | |
| planzliche Öle (zum Teil hydriert) | 225,65 | mg |
| Glycerol (85%) | | |
| Stabilisator E 420 | | |
| beta-Carotin als Farbstoff. | | |

9. Kombinationspräparat nach einem der Ansprüche 1 bis 8, dadurch gekennzeichnet, daß die Wirkstoffkombination den Bestandteil Selen bzw. Selen-Hefe umfaßt, daß das Selen bzw. die Selen-Hefe mit einem Anteil von bis zu 5 Gew.-% der gesamten Wirkstoffkombination vorgesehen ist, daß Wirkstoffkombination als Bestandteil ein Thymus-Extrakt umfaßt und daß es sich bei dem Thymus-Extrakt um ein Extrakt aus Rinderthymus oder um synthetisch hergestellte Thymus-Peptide handelt, wobei das Thymus-Extrakt vorzugsweise mit einem Gewichtsanteil von bis zu 50 Gew.-% der gesamten Wirkstoffkombination vorgesehen ist oder wobei die Thymus-Peptide mit einem Gewichtsanteil von bis zu 5 Gew.- % der gesamten Wirkstoffkombination vorgesehen sind.

10. Kombinationspräparat nach einem der Ansprüche 1 bis 9, dadurch gekennzeichnet, daß die Wirkstoffkombination den Bestandteil Melatonin umfaßt und daß der Wirkstoff Melatonin in einem Spektrum von 0,5 bis 30 mg vorgesehen ist.

11. Nicht therapeutische Verwendung des Kombinationspräparates nach einem der Ansprüche 1 bis 10 in Ergänzung zu einem topisch anwendbaren Haar- und ggf. Haut- und Nagelwuchsförderungsmittel, insbesondere zu einem thymushaltigen Therapeutikum.

## Claims

1. Combination preparation for stimulating the growth of hair and, optionally, the growth of skin and nails, as well as for preventing and eliminating the loss of hair with a combination of active ingredient containing vitamins, enzymes, and amino acids, c**haracterized in** that the combination of active ingredients is a component of a form of oral or even topical administration and contains approximately the following substances:
| | | |
|---|---|---|
| Provitamin A | 1.25 | wt.% |
| Vitamin B₁ | 0.27 | wt.% |
| Vitamins of the B₂ group | 5.25 | wt.% |
| Vitamin B₆ | 0.37 | wt.% |
| Vitamin B₁₂ | 0.001 | wt.% |
| Vitamin C | 15.61 | wt.% |
| Vitamin E | 2.5 | wt.% |
| Coenzyme Q 10 | 1.04 | wt.% |
| Methionine | 0.02 | wt.%, and |
| Cystine | 0.018 | wt.%. |

2. Combination preparation as in claim 1, characterized in that the vitamins of the B₂ group are mainly nicotinamide, pantothenic acid, and riboflavin and, optionally, folic acid and biotin and that the vitamins of the B₂ group are preferably contained in the combination of active ingredients in the following percentages:
| | | |
|---|---|---|
| Riboflavin | 0.35 | wt.% |
| Nicotinamide | 3.75 | wt.% |
| Folic acid | 0.09 | wt.% |
| Pantothenic acid | 1.04 | wt.% |
| Biotin | 0.02 | wt.% |
and whereas the panothenic acid is pantothenic acid from calcium pantothenate.

3. Combination preparation as in claim 1 or 2, characterized in that the provitamin A is at least primarily beta-carotene, that the vitamin B₁ is preferably present as thiamine nitrate, that the vitamin B₆ is preferably present as pyridoxine hydrochloride, that the vitamin B₁₂ is preferably present as cyanocobalamin and that the vitamin B₁₂ is present in a preparation with gelatin, whereas the preparation of vitamin B₁₂ and gelatin preferably amounts to about 1.04 wt.% of the combination of active ingredients.

4. Combination preparation as in one of claims 1-3, characterized in that the vitamin C is vitamin C from calcium ascorbate and that the vitamin E is present in the form of D, L-alpha-tocopherol acetate, whereas the methionine can be racemic methionine and whereas the cystine can be L-cystine.

5. Combination preparation as in one of claims 1-4, characterized in that the methionine and the cystine are contained in the combination of active ingredients as components of a milk protein, that the milk protein is lactalbumin 80 and that the lactalbumin 80 amounts to about 0.62 wt.% of the combination of active ingredients.

6. Combination preparation as in one of claims 1-5, characterized in that the combination of active ingredients is present in a formulation with linseed oil for preparing a form of administration and that the linseed oil is contained in the combination in an amount of 25.0 wt.%.

7. Combination preparation as in one of claims 1-6, characterized in that the combination of active ingredients is present in a formulation with modified and/or unmodified vegetable oils and/or in a formulation with an emulsifier, preferably emulsifier E 322, for preparing a form of administration.

8. Combination preparation as in one of claims 1-7, characterized in that the form of administration comprises a capsule which is present, if need be, in form of a preparation for receiving the combination of active ingredients, whereas the capsule could comprise at least gelatin and glycerol and/or a vegetable oil or fat and/or a stabilizer, in particular stabilizer 420, that the capsule is dyed, preferably with beta-carotene and that the filled capsule has a total weight of 480 mg, whereas the combination preparation is preferably composed in terms of weight from the combination of active ingredients:
| | | |
|---|---|---|
| Beta-carotene | 6.0 | mg |
| Vitamin B1 as thiamine nitrate | 1.3 | mg |
| Vitamin B2 as riboflavin | 1.7 | mg |
| Nicotinamide | 18.0 | mg |
| Folic acid | 0.45 | mg |
| Pantothenic acid from calcium pantothenate | 5.0 | mg |
| Biotin | 0.1 | mg |
| Vitamin B6 as pyridoxine hydrochloride | 1.8 | mg |
| Vitamin B12 as cyanocobalamin 0.1% with gelatin | 5.0 | mg |
| Vitamin C from calcium ascorbate | 75.0 | mg |
| Vitamin E as D, L-alpha-toco-pherol Acetate | 12.0 | mg |
| Coenzyme Q 10 | 5.0 | mg |
| Lactalbumin 80 with 3.6% cystine and 4% methionine in 80% protein of the lactalbumin | 3.0 | mg, |
and the adjuvants:
| | | |
|---|---|---|
| Linseed oil | 120.0 | mg, as well as |
| Emulsifier E 322 | | |
| Gelatin | | |
| Vegetable oils (partially hydrogenated) | 225.65 | mg |
| Glycerol (85%) | | |
| Stabilizer E 420 | | |
| Beta-carotene as dye. | | |

9. Combination preparation as in one of claims 1-8, characterized in that the combination of active ingredients comprises the component selenium or selenium yeast, that the selenium or the selenium yeast is provided in an amount of up to 5 wt.% of the total combination of active ingredients, that the combination of active ingredients comprises a thymus extract as component and that the thymus extract is an extract from cattle thymus or is synthetically produced thymus peptides, whereas the thymus extract is preferably provided in an amount of up to 50 wt.% of the total combination of active ingredients or whereas the thymus peptides are provided in an amount of up to 5 wt.% of the total combination of active ingredients.

10. Combination preparation as in one of claims 1-9, characterized in that the combination of active ingredients comprises the component melatonin and that the active ingredient melatonin is provided in a spectrum from 0.5 to 30 mg.

11. Non-therapeutical use of the combination preparation as in one of claims 1-10 as a supplement to a topically applicable stimulant for the growth of hair and, optionally, the growth of skin and nails, in particular as a supplement to a thymus-containing therapeutical agent.

## Revendications

1. Préparation combinée pour stimuler la pousse des cheveux et éventuellement celle des ongles et la régénération des tissus cutanés ainsi que pour empêcher ou lutter contre la chute des cheveux avec une combinaison de substances actives contenant des vitamines, des enzymes et des amino-acides,
caractérisée en ce que la combinaison de substances actives est une combinaison de forme de présentation à administration par voie orale ou transcutanée locale et contient sensiblement les substances suivantes:
| | | |
|---|---|---|
| provitamine A | 1,25 | % en poids |
| vitamine B₁ | 0,27 | % en poids |
| vitamines du groupe B₂ | 5,25 | % en poids |
| vitamine B₆ | 0,37 | % en poids |
| vitamine B₁₂ | 0,001 | % en poids |
| vitamine C | 15,61 | % en poids |
| vitamine E | 2,5 | % en poids |
| coenzyme Q 10 | 1,04 | % en poids |
| méthionine | 0.02 | % en poids et |
| cystine | 0,018 | % en poids |

2. Préparation combinée suivant la revendication 1, caractérisée en ce que les vitamines du groupe B₂ sont essentiellement de la nicotinamide, de l'acide pantothénique et de la riboflavine et le cas échéant de l'acide folique et de la biotine et en ce que les vitamines du groupe B₂ sont de préférence présentes dans les proportions suivantes dans la combinaison de substances actives:
| | | |
|---|---|---|
| riboflavine | 0,35 | % en poids |
| nicotinamide | 3,75 | % en poids |
| acide folique | 0,09 | % en poids |
| acide pantothénique | 1,04 | % en poids |
| biotine | 0,02 | % en poids |
et où l'acide pantothénique est un acide pantothénique obtenu à partir de pantothénate de calcium.

3. Préparation combinée suivant la revendication 1 ou 2, caractérisée en ce la provitamine A est au moins essentiellement de la bêta-carrotine, en ce que la vitamine B₁ est de préférence sous forme de nitrate de thiamine, en ce que la vitamine B₆ est de préférence sous forme d'hydrochlorure de pyridoxine, en ce que la vitamine B₁₂ est de préférence sous forme de cyano-cobalamine et en ce que la vitamine B₁₂ est présente dans une préparation avec de la gélatine, cette préparation de vitamine B₁₂ et de gélatine constituant de préférence environ 1,04 % en poids de la combinaison de substances actives.

4. Préparation combinée suivant l'une quelconque des revendications de 1 à 3, caractérisée en ce que la vitamine C est une vitamine C à base d'ascorbate de calcium et en ce que la vitamine E est sous forme de D,L-alpha-tocophérolacétate, la méthionine pouvant être une méthionine racémique et la cystine pouvant être de la L-cystine.

5. Préparation combinée suivant l'une quelconque des revendications de 1 à 4, caractérisée en ce que la méthionine et la cystine sont contenues dans la combinaison de substances actives sous forme de composants d une protéine de lait, en ce que la protéine de lait est la lactalbumine 80 et en ce que la lactalbumine 80 constitue environ 0,62 % en poids de la combinaison de substances actives.

6. Préparation combinée suivant l'une quelconque des revendications de 1 à 5, caractérisée en ce que la combinaison de substances actives est sous forme d'une préparation avec de l'huile de lin, et en ce que l'huile de lin est contenue dans la préparation combinée dans une proportion de 25,0 % en poids.

7. Préparation combinée suivant l'une quelconque des revendications de 1 à 6, caractérisée en ce que la combinaison de substances actives qui est à la base de la préparation est présente sous forme de préparation avec des huiles végétales modifiées et/ou non modifiées et/ou dans une préparation avec un émulsifiant, de préférence l'émulsifiant E 322.

8. Préparation combinée suivant l'une quelconque des revendications de 1 à 7, caractérisée en ce qu'elle est présentée sous forme d'une capsule contenant la combinaison de substances actives le cas échéant sous forme de préparation, où la capsule peut comprendre au moins de la gélatine et du glycérol ainsi qu'une huile ou une graisse végétale et/ou un stabilisateur, notamment le stabilisateur 420, en ce que la capsule est colorée de préférence avec de la bêta-carotine et en ce que la capsule remplie présente un poids total de 480 mg, où la préparation combinée est, en poids, composée de préférence de la combinaison de substances actives:
| | | |
|---|---|---|
| bêta-carotine | 6,0 | mg, |
| vitamine B₁ sous forme de nitrate de thiamine | 1,3 | mg, |
| vitamine B₂ sous forme de riboflavine | 1,7 | mg, |
| nicotinamide | 18,0 | mg, |
| acide folique | 0,45 | mg, |
| acide pantothénique à base de pantothénate de calcium | 5,0 | mg, |
| biotine | 0,1 | mg, |
| vitamine B₆ sous forme de hydrochlorure de pyridoxine | 1,8 | mg, |
| vitamine B₆, cyano-cobalamine à 1 % avec de la gélatine | 5,0 | mg, |
| vitamine C à base d'ascorbate de calcium | 75,0 | mg, |
| vitamine E à base de D,L-alpha-tocophérolacétate | 12,0 | mg, |
| coenzyme Q 10 | 5,0 | mg, |
| lactalbumine 80 avec 3,6 % de cystine et 4 % de méthionine dans 80 % de protéine de lactalbumine | 3,0 | mg, |
et avec les adjuvants:
| | | |
|---|---|---|
| huile de lin | 120,0 | mg ainsi que |
| émulsifiant E 322 | | |
| gélatine | | |
| huiles végétales (en partie hydrogénées) | 225,65 | mg |
| glycérol (85 %) | | |
| stabilisateur E 420 | | |
| bêta-carotine en tant que colorant | | |

9. Préparation combinée suivant l'une quelconque des revendications de 1 à 8, caractérisée en ce que la combinaison de substances actives comprend le composant sélénium ou levure de sélénium, en ce que le sélénium ou la levure de sélénium est prévu dans une proportion jusqu'à 5 % en poids de la combinaison de substances actives globale, en ce que la combinaison de substances actives comprend en tant que composant un extrait de thymus et ce qu'il s'agit en l'occurrence d'un extrait de thymus de bovin ou de peptides de thymus préparées par voie synthétique, où l'extrait de thymus est prévu de préférence dans une proportion en poids jusqu'à 50 % de la combinaison de substances actives globale, ou bien où les peptides de thymus sont prévues dans une proportion en poids jusqu'à 5 % de la combinaison de substances actives globale.

10. Préparation combinée suivant l'une quelconque des revendications de 1 à 9, caractérisée en ce que la combinaison de substances actives comprend le composant mélatonine, et en ce que le composant mélatonine est prévu dans une fourchette de 0,5 à 30 mg.

11. Utilisation d'une préparation combinée suivant l'une quelconque des revendications 1 à 10 en complément à un produit favorisant la pousse des cheveux et éventuellement celle des ongles et la régénération des tissus cutanés, notamment en complément à une thérapeutique à teneur en thymus.
